Europäisches Patentamt

European Patent Office

Office européen des brevets

(11) Veröffentlichungsnummer: **0 287 767 B1**

# (12) EUROPÄISCHE PATENTSCHRIFT

(45) Veröffentlichungstag der Patentschrift: **24.07.91**

(21) Anmeldenummer: **88102356.8**

(22) Anmeldetag: **18.02.88**

(51) Int. Cl.⁵: **A61F 13/15**

(54) **Wegwerfwindel.**

(30) Priorität: **23.03.87 DE 3709125**

(43) Veröffentlichungstag der Anmeldung:
**26.10.88 Patentblatt 88/43**

(45) Bekanntmachung des Hinweises auf die
Patenterteilung:
**24.07.91 Patentblatt 91/30**

(84) Benannte Vertragsstaaten:
**AT BE CH DE ES FR GB GR IT LI LU NL SE**

(56) Entgegenhaltungen:
**EP-A- 0 009 278**   **EP-A- 0 080 647**
**DE-A- 3 022 916**   **FR-A- 2 534 781**
**US-A- 3 844 288**   **US-A- 3 867 940**

(73) Patentinhaber: **KOESTER KG**
**Industriestrasse 2**
**W-8602 Altendorf(DE)**

(72) Erfinder: **Lemke, Wolfgang**
**Wellhöferstrasse 15**
**W-8520 Erlangen(DE)**

(74) Vertreter: **Schneck, Herbert, Dipl.-Phys., Dr. et al**
**Rau & Schneck Patentanwälte Königstrasse 2**
**W-8500 Nürnberg 1(DE)**

## Beschreibung

Die Erfindung richtet sich auf eine Wegwerfwindel umfassend eine feuchtigkeitsundurchlässige äußere Kunststoff-Folie, welche an der Innenseite mit einem saugfähigen Polster versehen ist, wobei an den seitlichen Kanten eines Rückenteils Klebebänder befestigt sind, welche beim Anlegen der Windel mit der Außenseite eines Vorderteils lösbar verklebt werden können, wobei die äußere Kunststoff-Folie im Klebebereich des Vorderteils an der Innenseite verstärkt ist.

Eine derartige Wegwerfwindel ist z.B. aus der DE-A-30 22 916 bekannt. Eine Verstärkung der äußeren Kunststoff-Folie der Windel im Klebebereich ist deshalb wünschenswert, weil bei zeitgemäßen Wegwerfwindeln ein Öffnen und Wiederverschließen der Klebeverbindung möglich sein soll. Da andererseits die äußere Kunststoff-Folie eine geringe Eigensteifigkeit und Zähigkeit aufweisen soll, um eine hautsympathische Ausgestaltung und einen hohen Tragekomfort zu ermöglichen, besteht die Gefahr, daß beim Abziehen der Klebebänder diese äußere Kunststoff-Folie ausgerissen oder zumindest so gedehnt wird, daß ein zuverlässiges und haltbares Wiederverschließen nicht möglich ist.

Hierzu ist es aus der DE-A-30 22 916 bekannt, auf eine Folie eine flächige Klebstoffbeschichtung aufzubringen. Das Aufbringen eines Klebstoffes ist aber herstellungstechnisch aufgrund von dessen Konsistenz problematisch und muß im intermittierenden Betrieb mit verhältnismäßig langen Taktzeiten erfolgen, so daß die Anwendung zur Herstellung eines knapp kalkulierten Massenprodukts wirtschaftlich nicht vertretbar ist.

Bei einer aus der EP-A-0 080 647 bekannten Lösung ging man davon aus, daß eine Verstärkung, insbesondere in Form eines Folienbandes, an der Außenseite im Klebebereich angebracht sein müsse und im Vergleich zur Außenseite der äußeren Kunststoff-Folie eine glatte Oberfläche aufweisen müsse, um das wiederholte Anbringen und Lösen des Klebeverschlusses zu ermöglichen.

Hierdurch wird aber in Kauf genommen, daß bei der Aufbringung der Verstärkungsfolie herstellungstechnisch ein relativ großer Aufwand getrieben werden muß, weil die exakte Positionierung dieser Folie das optische Erscheinungsbild des fertigen Produkts wesentlich mitbestimmt. Darüber hinaus wird gerade in einem so wichtigen Bereich, wie beim Bauchbereich des Kindes, durch ein glattes, eigensteifes und damit wenig hautfreundliches Verstärkungsband in Kauf genommen, daß die vorteilhaften Eigenschaften einer relativ weichen und oberflächenrauhen, derzeit üblicherweise verwendeten äußeren Kunststoff-Folie verlorengehen.

Hiervon ausgehend liegt der Erfindung die Aufgabe zugrunde, eine gegebenenfalls auch mehrfache Wiederverschließbarkeit einer Windel der eingangs genannten Art bei kostengünstiger Herstellbarkeit zu gewährleisten.

Diese Aufgabe wird erfindungsgemäß durch das Kennzeichnendes Hauptanspruchs gelöst.

Vorzugsweise wird hierfür eine Kunststoff-Folie gewählt, welche eine hohe Reißfestigkeit und geringe Dehnbarkeit aufweist. Überraschenderweise hat sich gezeigt, daß im Verbund mit der äußeren Kunststoff-Folie hierdurch eine hohe Festigkeit erreicht wird, welche auch ein wiederholtes Lösen der Klebebänder ermöglicht. Durch die erfindungsgemäße Ausgestaltung wird das einheitliche äußere Erscheinungsbild einer derartigen Windel nicht beeinträchtigt und vor allem bleibt eine derartige Windel auch im Bauchbereich des Kindes tragefreundlich und angenehm. Demgegenüber ist die Innenseite der äußeren Kunststoff-Folie ohnehin durch Polsterungen bedeckt, so daß dort das Anbringen einer verhältnismäßig steifen Verstärkungsfolie nicht als unangenehm empfunden wird.

Vorteilhafterweise erfolgt die Verstärkung auf der Innenseite in Form wenigstens einer aufkaschierten zusätzlichen Folienschicht. Günstigerweise ist die Folie als Polypropylenfolie ausgebildet. Eine derartige Polypropylenfolie weist eine besonders hohe Dehnungsfestigkeit auf. Dabei kann vorgesehen sein, daß die Folie in Richtung quer zur Wegwerfwindel verstreckt ist, wobei die größte Festigkeit erzielt wird, wenn die Folie biaxial verstreckt ist.

Durch eine derartige besonders haltbare, an der Innenseite der Windel angebrachte Verstärkungsfolie wird nicht nur das angestrebte Ziel einer Verstärkung bei hoher Herstellungsgeschwindigkeit erreicht, sondern es wird auch verhindert, daß das Kind - wie bei herkömmlicherweise außen angebrachten Verstärkungsfolien - die Folie abziehen und verschlucken kann.

Bei einer erfindungsgemäßen Ausgestaltung ist vorgesehen, daß beiderseits am Vorderteil zwei voneinander getrennte, verstärkte Bereiche ausgebildet sind. Demnach wird eine Verstärkung also nur dort vorgenommen, wo in der Regel tatsächlich die Klebebänder angebracht werden. Dies bedeutet, daß insbesondere der Mittelbereich nicht verstärkt wird, so daß sich dort eine gute Elastizität und Schmiegsamkeit ergibt, wobei darüber hinaus noch der Aufwand an Verstärkungsmaterial auf ein Minimum reduziert wird.

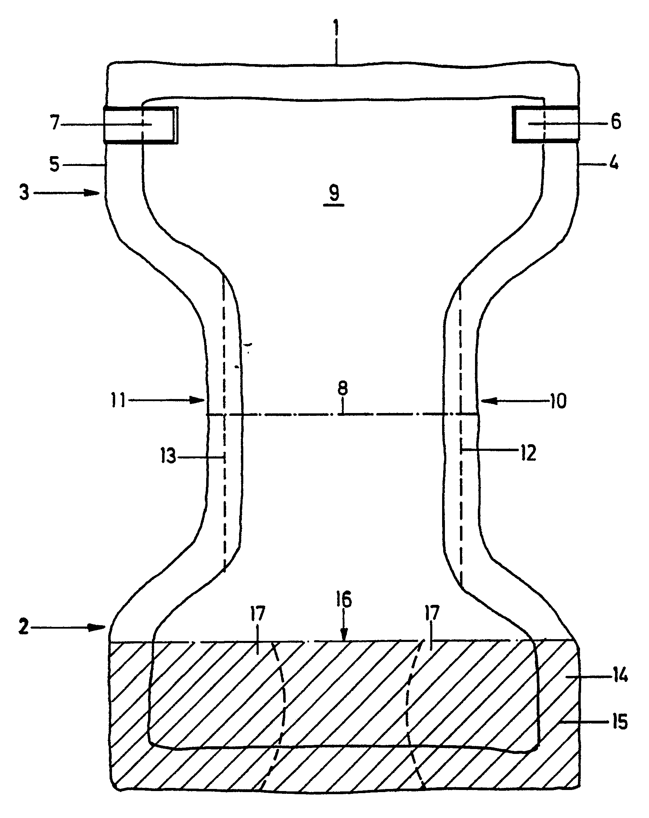

Weitere Merkmale, Vorteile und Einzelheiten der Erfindung ergeben sich aus der folgenden Beschreibung einer bevorzugten Ausführungsform anhand der Zeichnung. Diese zeigt eine schematische Darstellung einer erfindungsgemäßen Wegwerfwindel im flachen, aufgeklappten Zustand.

In der Zeichnung ist eine Wegwerfwindel im aufgeklappten Zustand derart dargestellt, daß die Windelinnenseite sichtbar ist. Eine derartige Windel umfaßt einen Zuschnitt aus einer äußeren Kunststoff-Folie 1, welche im wesentlichen ein Vorderteil 2 und ein Rückenteil 3 umfaßt. Im Bereich der seitlichen Außenkanten 4,5 des Rückenteils 3 sind Klebestreifen 6,7 angebracht, welche beim Anlegen der Windel von der äußeren Innenseite, d.h. von einem dort angebrachten, sogenannten Release Tape, gelöst werden. Das Vorderteil 2 wird dann um den strichpunktiert eingezeichneten Faltbereich 8 herum nach oben geklappt und die Klebestreifen 6,7 werden in an sich bekannter Weise an der äußeren Kunststoff-Folie 1 am Vorderteil 2 befestigt.

Die Innenseite der äußeren Kunststoff-Folie 1 ist mit einem saugfähigen Polster 9 versehen, welches in an sich bekannter Weise ausgestaltet und dementsprechend nur schematisch angedeutet ist. Im Bereich der Beinausschnitte 1o,11 sind ebenfalls nur schematisch angedeutete Elastikzüge 12,13 eingearbeitet, welche für eine dichte Anlage der Windel um die Beine des Kindes herum sorgen.

An der Innenseite 14 der äußeren Kunststoff-Folie 1 ist in dem Bereich 15 ( in der Zeichnung schraffiert dargestellt), innerhalb dessen an der Außenseite der Folie 1 die Klebestreifen 6 zur Anlage kommen, eine Verstärkungsfolie 16 aufkaschiert, welche gegenüber der äußeren Folie 1 eine höhere Reißfestigkeit aufweist.

Die Verstärkungsfolie 16 kann auch, wie gestrichelt bei 17 jeweils angedeutet, lediglich in Form von zwei voneinander getrennten Abschnitten beiderseits der äußeren Randbereiche angebracht werden.

## Patentansprüche

1. Wegwerfwindel umfassend eine feuchtigkeitsundurchlässige äußere Kunststoff-Folie, welche an der Innenseite mit einem saugfähigen Polster versehen ist, wobei an den seitlichen Kanten eines Rückenteils Klebebänder befestigt sind, welche beim Anlegen der Windel mit der Außenseite eines Vorderteils lösbar verklebt werden können, wobei die äußere Kunststoff-Folie im Klebebereich des Vorderteils an der Innenseite verstärkt ist, wobei die Verstärkung in Form wenigstens einer glatten Folie (16) an der Innenseite (14) der Kunststoff-Folie (1) des Vorderteils (2) angebracht ist, dadurch gekennzeichnet, daß die Folie mit drucksensitivem Klebstoff aufgebracht ist.

2. Wegwerfwindel nach Anspruch 1, dadurch gekennzeichnet daß die Folie (16) eine Polypropylenfolie ist.

3. Wegwerfwindel nach Anspruch 2, dadurch gekennzeichnet daß die Polypropylenfolie (16) in Richtung quer zur Wegwerfwindel verstreckt ist.

4. Wegwerfwindel nach Anspruch 3, dadurch gekennzeichnet daß die Polypropylenfolie biaxial verstreckt ist.

5. Wegwerfwindel nach Anspruch 1, dadurch gekennzeichnet daß beiderseits am Vorderteil (2) zwei voneinander getrennt verstärkte Bereiche (17) ausgebildet sind.

## Claims

1. Disposable baby's nappy comprising a non-moisture permeable outer plastic foil which is provided on the inside with absorbent padding, there being secured to the side edges of a rear part adhesive tapes which, on the application of the nappy to a child, are detachably stuck to the outside of a front part, the outer plastic foil being reinforced in the adhesive region of the front part, the reinforcement being applied in the form of at least one smooth foil (16) on the inside (14) of the plastic foil (1) of the front part (2), characterized in that the foil (16) is applied with a pressure-sensitive adhesive.

2. Disposable baby's nappy according to claim 1, characterized in that the foil (16) is a polypropylene foil.

3. Disposable baby's nappy according to claim 2, characterized in that the polypropylene foil (16) is oriented in cross-direction of the disposable nappy.

4. Disposable baby's nappy according to claim 3, characterized in that the polypropylene foil is biaxially oriented.

5. Disposable baby's nappy according to claim 1, characterized in that two reinforced regions (17) separate from each other are formed on both sides on the front part (2).

## Revendications

1. Couche à jeter comportant une feuille extérieure en matière plastique, imperméable à l'humidité et pourvue sur la face intérieure d'un matelas absorbant, des bandes collantes étant fixées sur les bords latéraux d'une partie dorsale et pouvant être collées de manière amovible sur la face extérieure d'une partie avant lors de la mise en place de la couche, la feuille extérieure en matière plastique étant renforcée sur le côté intérieur dans la zone de collage de la partie avant, le renfort se présentant sous la forme d'au moins une feuille lisse (16) fixée sur la face intérieure (14) de la feuille en matière plastique (1) de la partie avant (2), caractérisée en ce que la feuille (16) est appliquée à l'aide d'une colle sensible à la pression.

2. Couche à jeter selon la revendication 1, caractérisée en ce que la feuille (16) est une feuille de polypropylène.

3. Couche à jeter selon la revendication 2, caractérisée en ce que la feuille de polypropylène (16) est étirée dans la direction transversale de la couche à jeter.

4. Couche à jeter selon la revendication 3, caractérisée en ce que la feuille de polypropylène est étirée de manière bi-axiale.

5. Couche à jeter selon la revendication 1, caractérisée en ce que deux zones renforcées (17), séparées l'une de l'autre, sont réalisées chacune d'un côté de la partie avant (2).